# EUROPEAN PATENT APPLICATION

(11) **EP 4 375 290 A1**
(43) Date of publication of application: **29.05.2024**
(21) Application number: 22846202.4
(22) Date of filing: 19.07.2022
(51) Int. Cl.: C07K 14/005, C12N 15/82, A61K 39/215, A61P 31/14

(54) **PLANT-BASED COVID-19 VARIANT RECOMBINANT SPIKE PROTEIN EXPRESSION VECTOR AND RECOMBINANT PROTEIN USING SAME**

(30) Priority: 19.07.2021 KR 20210093990
(71) Applicant: Bioapplications Inc., Pohang-si, Gyeongsangbuk-do 37668 (KR)
(72) Inventor: SOHN, Eun-Ju, Pohang-si, Gyeongsangbuk-do 37668 (KR); CHOI, Bo-Hwa, Pohang-si, Gyeongsangbuk-do 37655 (KR); KANG, Hyangju, Pohang-si, Gyeongsangbuk-do 37780 (KR); MIN, Kyungmin, Pohang-si, Gyeongsangbuk-do 37831 (KR); PARK, Minhee, Pohang-si, Gyeongsangbuk-do 37780 (KR); KIM, Nam Hyung, Pohang-si, Gyeongsangbuk-do 37560 (KR); KIM, Daniel, Pohang-si, Gyeongsangbuk-do 37604 (KR); LEE, Yoo-Kyoung, Cheongju-si, Chungcheongbuk-do 28159 (KR); KIM, Byung-Chul, Cheongju-si, Chungcheongbuk-do 28159 (KR); LIM, Heeji, Cheongju-si, Chungcheongbuk-do 28159 (KR); JANG, Sundong, Cheongju-si, Chungcheongbuk-do 28159 (KR); KIM, Kwangwook, Cheongju-si, Chungcheongbuk-do 28159 (KR); KIM, Soo Ji, Cheongju-si, Chungcheongbuk-do 28159 (KR); KIM, Dokeun, Cheongju-si, Chungcheongbuk-do 28159 (KR); KIM, You-Jin, Cheongju-si, Chungcheongbuk-do 28159 (KR); LEE, Jung-Ah, Cheongju-si, Chungcheongbuk-do 28159 (KR); PARK, Woo-Jung, Cheongju-si, Chungcheongbuk-do 28159 (KR)
(74) Representative: Weickmann & Weickmann PartmbB
(86) International application number: PCT/KR2022/010552
(87) International publication number: WO 2023/003332

(57) **Abstract**

The present invention relates to a plant-based COVID-19 variant recombinant spike protein expression vector, and a recombinant protein using the expression vector, wherein a plant expression system is used such that the recombinant protein does not have the disadvantages of animal-derived recombinant proteins and exhibits an excellent SARS coronavirus 2 prevention and treatment effect, and thus can be effectively utilized as a safe composition for preventing and treating COVID-19.

## Description

### [Technical Field]

The present invention relates to a plant-based COVID-19 variant recombinant spike protein expression vector and a recombinant protein using the expression vector.

This application claims priority based on Korean Patent Application No. 10-2021-0093990, filed on July 19, 2021, and Korean Patent Application No. 10-2022-0088990, filed on July 19, 2022, and all contents disclosed in the specifications and drawings of the applications are incorporated into this application.

### [Background Art]

SARS-CoV-2 is a pathogen of coronavirus infection-19 (COVID-19), which was first discovered on December 12, 2019, and human-to-human infection was confirmed. SARS-CoV-2 is known to strongly attach to the surface of host cells by forming a bond with the angiotensin converting enzyme 2 (ACE2) receptor through the receptor-binding domain (RBD) located at positions 331 to 524 of the full-length amino acid sequence of the spike protein.

The main symptoms of COVID-19 are respiratory symptoms such as fever, cough, sore throat and shortness of breath, and depending on the patient, various symptoms such as headache, muscle pain, hemoptysis and nausea, chills, chest pain, diarrhea, loss of taste and smell, loss of lung function and the like appear. Healthy adults are likely to recover over time, but it can be fatal if someone with low immune function, such as the elderly or those with underlying diseases, becomes infected. Some may progress to post-infectious acute respiratory distress syndrome (ARDS), acute lung injury, septic shock, acute kidney injury and the like, and in severe cases, it has been reported that it may lead to death.

However, in spite of the high transmissibility and high risk of SARS-coronavirus-2, only a few verified vaccines or treatments have been developed to prevent or treat SARS-coronavirus infectious diseases. Therefore, the recurrence of SARS-CoV and MERS-CoV infections, which have been a major global issue, or a new pandemic phenomenon may occur at any time, and the situation is that the rapid production of vaccines for viruses is required.

Meanwhile, as a method for producing useful physiologically active substances that can replace animal cells or microorganisms, the protein production method using a plant expression system has recently received great attention. In plant cells, modification after translation into protein is very similar to that in animal cells, and thus, multimeric proteins can be accurately produced, and in the case of glycoproteins, it has the advantage of being able to obtain a protein having the same form of modification as the original protein, because it can be separated in the form of glycosylation.

Furthermore, in the case of producing useful physiologically active substances using plants, various sources of contamination that may occur during protein production from animal cells or microorganisms can be excluded at the source, and when the demand for the corresponding useful substances soars, since it is absolutely advantageous compared to existing animal cell systems in terms of equipment technology and cost required for mass production, it has the advantage of being able to mass-produce in a short period of time and at low cost.

However, almost no composition for preventing or treating SARS-coronavirus 2 using a recombinant protein prepared from plants has been reported.

### [Disclosure]

### [Technical Problem]

The inventors of the present invention completed the present invention by conducting research to prepare a recombinant protein that exhibits excellent preventive and therapeutic effects of SARS-coronavirus 2 without the disadvantages of animal-derived recombinant proteins by using a plant expression system.

An object of the present invention is to provide an expression vector for an antigen protein for preventing or treating COVID-19, wherein in a polynucleotide encoding an S 1 subunit of the spike glycoprotein represented by the amino acid sequence of SEQ ID NO: 1, the S1 subunit has a mutation at one or more positions selected from the group consisting of positions 417, 484, 501 and 614 from the N-terminus of the amino acid sequence.

Another object of the present invention is to provide a transformant, which is transformed with the recombinant vector.

Still another object of the present invention is to provide a method for preparing an antigen protein for preventing or treating COVID-19, including the steps of transforming the recombinant vector into a plant; and separating and purifying a recombinant antigen from the plant.

Still another object of the present invention is to provide a composition for preventing or treating COVID-19, including the antigen produced by the method as an active ingredient.

Still another object of the present invention is to provide a kit for preventing or treating COVID-19, including the antigen produced by the method as an active ingredient.

Still another object of the present invention is to provide a method for preventing or treating COVID-19, including the step of administering a composition including the antigen produced by the method as an active ingredient to a non-human animal.

Still another object of the present invention is to provide a vaccine composition for preventing or treating COVID-19, including the vector or antigen produced by the method as an active ingredient.

In addition, another object of the present invention is to provide the use of a composition including the vector or the antigen produced by the method as an active ingredient in the prevention or treatment of COVID-19.

In addition, still another object of the present invention is to provide the use of a composition including the vector or the antigen produced by the method as an active ingredient in the preparation of a medicament for treating COVID-19.

However, the technical problems to be achieved by the present invention are not limited to the above-mentioned problems, and other problems that are not mentioned will be clearly understood by those skilled in the art from the description below.

### [Technical Solution]

In order to achieve the above-described objects, the present invention provides a recombinant vector for an antigen protein for preventing or treating COVID-19, wherein in a polynucleotide encoding an S1 subunit of the spike glycoprotein represented by the amino acid sequence of SEQ ID NO: 1, the S1 subunit has a mutation at one or more positions selected from the group consisting of positions 417, 484, 501 and 614 from the N-terminus of the amino acid sequence.

In an embodiment of the present invention, the recombinant vector may further include a polynucleotide encoding an S2 subunit of the spike glycoprotein represented by the amino acid sequence of SEQ ID NO: 2, and the S2 subunit may have a mutation at one or more positions selected from the group consisting of positions 942, 986 and 987 from the N-terminus of the amino acid sequence, but the present invention is not limited thereto.

In another embodiment of the present invention, the mutation may be any one or more mutations selected from the group consisting of K417N, E484K, N501Y and D614G, but the present invention is not limited thereto.

In still another embodiment of the present invention, the mutation may be any one or more mutations selected from the group consisting of A942P, K986P and V987P, but the present invention is not limited thereto.

In still another embodiment of the present invention, the recombinant vector may further include a polynucleotide encoding the new chaperone binding protein (New BiP; NB) protein, but the present invention is not limited thereto.

In still another embodiment of the present invention, the recombinant vector may further include a polynucleotide encoding the foldon (Fd) protein, but the present invention is not limited thereto.

In still another embodiment of the present invention, the recombinant vector may further include a polynucleotide encoding the HDEL protein, but the present invention is not limited thereto.

In still another embodiment of the present invention, the recombinant vector may further include a polynucleotide encoding an antibody in which the furin cleavage site (PRRA) between the S1 subunit and the S2 subunit is deleted, but the present invention is not limited thereto.

In still another embodiment of the present invention, the recombinant vector may be expressed in a plant, but the present invention is not limited thereto.

The present invention provides a transformant, which is transformed with the recombinant vector.

In still another embodiment of the present invention, the transformant may be a plant, but the present invention is not limited thereto.

The present invention provides a method for preparing an antigen protein for preventing or treating COVID-19, including the steps of transforming the recombinant vector into a plant; and separating and purifying a recombinant antigen from the plant.

The present invention provides a composition for preventing or treating COVID-19, including the antigen produced by the method as an active ingredient.

The present invention provides a kit for preventing or treating COVID-19, including a composition including the antigen produced by the method as an active ingredient and instructions.

The present invention provides a method for preventing or treating COVID-19, including the step of administering a composition including the antigen produced by the method as an active ingredient to a non-human animal.

The present invention provides a vaccine composition for preventing or treating COVID-19, including the vector or the antigen produced by the method as an active ingredient.

In addition, the present invention provides the use of a composition including the vector or the antigen produced by the method as an active ingredient in the prevention or treatment of COVID-19.

In addition, the present invention provides the use of a composition including the vector or the antigen produced by the method as an active ingredient in the preparation of a medicament for treating COVID-19.

### [Advantageous Effects]

According to the plant-based COVID-19 variant recombinant spike protein expression vector and the recombinant protein using the expression vector, a plant expression system is used such that the recombinant protein does not have the disadvantages of animal-derived recombinant proteins and exhibits an excellent SARS coronavirus 2 prevention and treatment effect, and thus can be effectively utilized as a safe composition for preventing and treating COVID-19.

### [Description of Drawings]

FIG. 1 is a cleavage map showing the structure of the COVID-19 variant recombinant spike protein expression vector.
FIG. 2 is a graph showing the ACE2 binding activity of the recombinant antigen (S(rBeta3P)) produced by the COVID-19 variant recombinant spike protein expression vector.
FIG. 3 is a graph showing the schematic diagram of an animal experiment design for an anti-Spike IgG ELISA experiment of the recombinant antigen (S(rBeta3P)) produced by a COVID-19 variant recombinant spike protein expression vector.
FIG. 4 is a graph showing the PRNT test results for the Wuhan virus of the recombinant antigen (S(rBeta3P)) produced by a COVID-19 variant recombinant spike protein expression vector.
FIG. 5 is a graph showing the PRNT test results for the beta-mutant virus of the recombinant antigen (S(rBeta3P)) produced by a COVID-19 variant recombinant spike protein expression vector.
FIG. 6 is a graph showing the PRNT test results for the delta mutant virus of the recombinant antigen (S(rBeta3P)) produced by a COVID-19 variant recombinant spike protein expression vector.
FIG. 7 is a graph showing the PRNT test results for the Omicron mutant virus of the recombinant antigen (S(rBeta3P)) produced by a COVID-19 variant recombinant spike protein expression vector.
FIG. 8 is a graph showing the anti-Spike IgG ELISA test results of the recombinant antigen (S(rBeta3P)) produced by a COVID-19 variant recombinant spike protein expression vector.
FIG. 9 is a graph showing the schematic diagram of an animal experiment design for an intracellular cytokine staining experiment of the recombinant antigen (S(rBeta3P)) produced by a COVID-19 variant recombinant spike protein expression vector.
FIG. 10(a) is a graph showing the expression level of INF-γ in CD8⁺ T cells after stimulation with the inoculum antigen protein for intracellular cytokine staining experiments on the recombinant antigen (S(rBeta3P)) produced by a COVID-19 variant recombinant spike protein expression vector.
FIG. 10(b) is a graph showing the expression level of INF-γ in CD8⁺ T cells after stimulation with a CD8-specific peptide pool for intracellular cytokine staining experiments on the recombinant antigen (S(rBeta3P)) produced by a COVID-19 variant recombinant spike protein expression vector.
FIG. 11(a) is a graph showing the expression level of TNF-α in CD8⁺ T cells after stimulation with the inoculum antigen protein for intracellular cytokine staining experiments on the recombinant antigen (S(rBeta3P)) produced by a COVID-19 variant recombinant spike protein expression vector.
FIG. 11(b) is a graph showing the expression level of TNF-α in CD8⁺ T cells after stimulation with a CD8-specific peptide pool for intracellular cytokine staining experiments on the recombinant antigen (S(rBeta3P)) produced by a COVID-19 variant recombinant spike protein expression vector.
FIG. 12(a) is a graph showing the expression level of INF-γ in CD4⁺ T cells after stimulation with the inoculum antigen protein for intracellular cytokine staining experiments on the recombinant antigen (S(rBeta3P)) produced by a COVID-19 variant recombinant spike protein expression vector.
FIG. 12(b) is a graph showing the expression level of INF-γ in CD4⁺ T cells after stimulation with a CD8-specific peptide pool for intracellular cytokine staining experiments on the recombinant antigen (S(rBeta3P)) produced by a COVID-19 variant recombinant spike protein expression vector.
FIG. 13(a) is a graph showing the expression level of TNF-α in CD4⁺ T cells after stimulation with the inoculum antigen protein for intracellular cytokine staining experiments on the recombinant antigen (S(rBeta3P)) produced by a COVID-19 variant recombinant spike protein expression vector.
FIG. 13(b) is a graph showing the expression level of TNF-α in CD4⁺ T cells after stimulation with a CD8-specific peptide pool for intracellular cytokine staining experiments on the recombinant antigen (S(rBeta3P)) produced by a COVID-19 variant recombinant spike protein expression vector.
FIG. 14(a) is a graph showing the expression level of INF-γ in splenocytes after stimulation with the inoculum antigen protein for ELISpot-cytokines experiments on the recombinant antigen (S(rBeta3P)) produced by a COVID-19 variant recombinant spike protein expression vector.
FIG. 14(b) is a graph showing the expression level of INF-γ in splenocytes after stimulation with a CD8-specific peptide pool for ELISpot-cytokines experiments on the recombinant antigen (S(rBeta3P)) produced by a COVID-19 variant recombinant spike protein expression vector.
FIG. 15(a) is a graph showing the expression level of IL-4⁺ in splenocytes after stimulation with the inoculum antigen protein for ELISpot-cytokines experiments for the recombinant antigen (S(rBeta3P)) produced by a COVID-19 variant recombinant spike protein expression vector.
FIG. 15(b) is a graph showing the expression level of IL-4⁺ in splenocytes after stimulation with a CD8-specific peptide pool for ELISpot-cytokines experiments on the recombinant antigen (S(rBeta3P)) produced by a COVID-19 variant recombinant spike protein expression vector.

### [Best Mode]

The present invention relates to a COVID-19 variant recombinant spike protein expression vector and a recombinant protein prepared therefrom, and specifically, the present invention relates to a recombinant protein which consists of NB protein-S1 protein-S2 protein-Fd polypeptide-HDEL peptide, wherein D614G mutation occurs at the spike of the S1 protein, K417N, E484K and N501Y mutations occur at the RBD of the S1 protein, and A942P, K986P and V987P mutations occur at the S2 protein, respectively, so as to have excellent immunogenicity.

As used herein, the term "coronavirus disease-19" is a severe respiratory syndrome caused by SARS-CoV-2.

The SARS-coronavirus 2 infection may include an infection caused by the SARS-corona mutant virus 2, and for example, the SARS-corona mutant virus 2 may be any one or more SARS-corona mutant virus 2 selected from the group consisting of alpha, beta, gamma, delta, epsilon, zeta, eta, theta, yota, kappa, lambda, mu and omicron corona viruses, but the present invention is not limited thereto.

As used herein, the term "recombinant expression vector" refers to a bacterial plasmid, phage, yeast plasmid, plant cell virus, mammalian cell virus or other vector. In general, any plasmid and vector may be used, provided that it is capable of replicating and stabilizing in the host. The recombinant expression vector of the present invention may preferably include a promoter, which is a transcription initiation factor to which RNA polymerase binds, an arbitrary operator sequence for regulating transcription, a sequence encoding a suitable mRNA ribosome binding site, a sequence for regulating the termination of transcription and translation, a terminator and the like.

The recombinant expression vector and expression vector including appropriate transcriptional/translational control signals may be constructed by methods well known to those skilled in the art. The methods include *in vitro* recombinant DNA technology, DNA synthesis technology, *in vivo* recombinant technology and the like.

In addition, it may further include a tag gene for increasing the production amount of a recombinant protein, a tag gene for maintaining the structural stability of the recombinant protein, a tag gene for easily isolating the recombinant protein, a selection marker gene such as an antibiotic-resistant gene for selecting a transgenic organism and the like, and representative examples of a tag for easy isolation include an Avi tag, a Calmodulin tag, a polyglutamate tag, an E tag, a FLAG tag, a HA tag, a His tag, an Myc tag, a S tag, a SBP tag, an IgG-Fc tag, a CTB tag, a Softag 1 tag, a Softag 3 tag, a Strep tag, a TC tag, a V5 tag, a VSV tag, an Xpress tag and the like, and representative examples of the selection marker gene include an herbicide resistance gene such as glyphosate or phosphinothricin, an antibiotic resistance gene such as kanamycin, G418, bleomycin, hygromycin and chloramphenicol, an aadA gene and the like, but the present invention is not limited thereto, and representative examples of the promoter include a pEMU promoter, a MAS promoter, a histone promoter, a Clp promoter, a 35S promoter derived from the cauliflower mosaic virus, a 19S RNA promoter derived from the cauliflower mosaic virus, an actin protein promoter of a plant, a ubiquitin protein promoter, a cytomegalovirus (CMV) promoter, a simian virus 40 (SV40) promoter, a respiratory syncytial virus (RSV) promoter, an elongation factor-1 alpha (EF-1α) promoter and the like, and representative examples of the terminator include nopaline synthase (NOS), a rice amylase RAmy1 A terminator, a phaseolin terminator, an Octopine gene terminator of *Agrobacterium tumefaciens*, an *E. coli* rmB1/B2 terminator and the like, but the type of added gene is not limited as long as it is a type used for the preparation of the existing recombinant protein.

A preferred example of the recombinant vector of the present invention is a Ti-plasmid vector which is capable of transferring a part of itself, the so-called T-region, into plant cells when it is present in a suitable host. Another type of Ti-plasmid vector is currently being used to transfer hybrid DNA sequences into plant cells or protoplasts from which new plants can be produced that properly integrate the hybrid DNA into the plant's genome. A particularly preferred form of the Ti-plasmid vector is the so-called binary vector as claimed in EP 0120 516 B1 and US Patent. No. 4,940,838. Other suitable vectors that can be used to introduce DNA according to the present invention into plant hosts include viral vectors, such as those that can be derived from double-stranded plant viruses (e.g., CaMV) and singlestranded viruses, gemini viruses and the like, and for example, it may be selected from incomplete plant viral vectors. The use of such vectors may be particularly advantageous when properly transforming a plant host is difficult.

As used herein, the term "antigen" refers to all substances that cause an immune response in the body, and is preferably a virus, chemical substance, bacterium, pollen, cancer cell, shrimp and the like, or some peptide or protein thereof, or a substance that can cause an immune response.

In the present invention, it may include a recombinant antigen protein in which at least one amino acid of the polypeptide is mutated. The mutation includes amino acid substitution, deletion, addition and the like, but preferably includes substitution. Substitution of the amino acid sequence of the polypeptide may increase antigen yield. The substitution means that an existing amino acid sequence is replaced with another amino acid sequence. At least one amino acid may be substituted, and preferably, 1 to 11 amino acids may be substituted, and for example, 2 to 10 amino acids may be substituted with other amino acids, and in another example, 9, 8, 7, 6, 5, 4, 3 or 2 amino acids may be substituted.

The present invention provides a recombinant vector for an antigen protein for preventing or treating COVID-19, wherein in a polynucleotide encoding an S1 subunit of the spike glycoprotein represented by the amino acid sequence of SEQ ID NO: 1, the S1 subunit has a mutation at one or more positions selected from the group consisting of positions 417, 484, 501 and 614 from the N-terminus of the amino acid sequence.

In an exemplary embodiment of the present invention, the recombinant vector may further include a polynucleotide encoding an S2 subunit of the spike glycoprotein represented by the amino acid sequence of SEQ ID NO: 2, and the S2 subunit may have a mutation at one or more positions selected from the group consisting of positions 942, 986 and 987 from the N-terminus of the amino acid sequence, but the present invention is not limited thereto.

In an exemplary embodiment of the present invention, the mutation may be any one or more mutations selected from the group consisting of K417N, E484K, N501Y and D614G, but the present invention is not limited thereto.

In the present invention, the K417N, E484K, N501Y and D614G may mean that lysine (K) at position 417 from the N-terminus of the amino acid sequence of the S1 subunit of SEQ ID NO: 1 is substituted with asparagine (N), glutamic acid (E) at position 484 is substituted with lysine (K), asparagine (N) at position 501 is substituted with tyrosine (Y), and aspartic acid (D) at position 614 is substituted with glycine (G).

In an exemplary embodiment of the present invention, the mutation may be any one or more mutations selected from the group consisting of A942P, K986P and V987P, but the present invention is not limited thereto.

In the present invention, the A942P, K986P and V987P may mean that alanine (A) at position 942 from the N-terminus of the amino acid sequence of the S2 subunit of the spike glycoprotein represented by SEQ ID NO: 2 is substituted with proline (P), lysine (K) at position 986 is substituted with proline (P), and valine (V) at position 987 is substituted with proline (P).

In an exemplary embodiment of the present invention, the recombinant vector may further include a polynucleotide encoding the new chaperone binding protein (New BiP; NB) protein, but the present invention is not limited thereto.

In an exemplary embodiment of the present invention, the recombinant vector may further include a polynucleotide encoding the foldon (Fd) protein, but the present invention is not limited thereto.

In the present invention, a "foldon domain" may have any foldon sequence known to those skilled in the art. Preferably, it may include a foldon of bacteriophage T4 fibritin, and it is represented by the amino acid sequence of SEQ ID NO: 3. The spike protein on the surface of the virus forms a trimer, but the S ectodomain in which the transmembrane domain is removed does not stably form a trimer and may exist as a monomer or dimer. The foldon domain may induce the S ectodomain antigen to stably form a trimer, help to achieve a structure similar to S on the surface of the virus, and increase the size of the antigen and thereby increase antigenicity.

In an exemplary embodiment of the present invention, the recombinant vector may further include a polynucleotide encoding the HDEL protein, but the present invention is not limited thereto.

In an exemplary embodiment of the present invention, the recombinant vector may further include a polynucleotide encoding an antibody in which the furin cleavage site (PRRA) between the S1 subunit and the S2 subunit is deleted, but the present invention is limited thereto.

The recombinant vector according to the present invention may be constructed in the order of NB code sequence, S 1 code sequence, S2 code sequence, Fd code sequence, 7His code sequence and HDEL code sequence, but the present invention is not limited thereto.

In addition to the above sequences, one or more components that can be generally used by those skilled in the art may be added in order to improve the function, production yield, delivery power and the like of the recombinant antigen by the recombinant vector according to the present invention, and in this case, there is no limitation to the location of the added components, and the combination, order and the like of each component in the vector may be changed.

Further, in order to improve the function, production yield, delivery power and the like of the recombinant antigen by the recombinant vector according to the present invention, those skilled in the art may apply generally applicable methods such as mutation change, generation, addition and the like, even in this case, the combination and order of each component in the vector may be changed as necessary.

In an exemplary embodiment of the present invention, the recombinant vector may be expressed in a plant, but the present invention is not limited thereto.

In the present invention, a "plant" may be used without limitation as long as it is a plant which is capable of mass-producing the recombinant protein of the present invention, and it may be at least one dicotyledonous plant selected from the group consisting of *Arabidopsis,* soybean, tobacco, eggplant, red pepper, potato, tomato, Chinese cabbage, radish, cabbage, lettuce, peach, pear, strawberry, watermelon, melon, cucumber, carrot and celery; or may be selected from the group consisting of rice, barley, wheat, rye, corn, sorghum, oat and onion.

In addition, the present invention provides a transformant, which is transformed with the recombinant vector.

In the present invention, the transformant may be a plant, but the present invention is not limited thereto.

As used herein, the term "transformation" is a general term for changes in the genetic properties of organisms by injected DNA, and the term "transgenic organism" is a living organism that is prepared by injecting external genes by using molecular genetic methods, and preferably, it is a living organism that is transformed by the recombinant expression vector of the present invention, and the living organism is not limited as long as it is a living organism such as a microorganism, eukaryotic cell, insect, animal, plant and the like, and preferably, it may be *Escherichia coli*, *Salmonella, Bacillus,* yeast, animal cells, mice, rats, dogs, monkeys, pigs, horses, cows, *Agrobacterium tumefaciens*, plants and the like, but the present invention is not limited thereto. The transformant may be prepared by methods such as transformation, transfection, *Agrobacterium*-mediated transformation method, particle gun bombardment, sonication, electroporation, PEG (polyethylene glycol)-mediated transformation method and the like, and there is no limitation as long as it is a method for injecting the vector of the present invention.

In addition, the present invention provides a method for preparing a recombinant antigen protein for preventing or treating COVID-19, including the steps of culturing the transformant; and separating and purifying a recombinant antigen protein for preventing or treating COVID-19 from the transformant or a culture medium thereof.

In the case of transforming the vector of the present invention into a eukaryotic cell, yeast (*Saccharomyce cerevisiae*), insect cell, human cell (*e.g.,* CHO (Chinese hamster ovary) cell line, W138, BHK, COS-7, 293, HepG2, 3T3, RIN and MDCK cell lines), plant cell and the like may be used as a host cell, and preferably, it may be *Agrobacterium.*

When the host cell is a prokaryotic cell, the method for delivering the vector of the present invention into the host cells may be performed by the CaCl₂ method, the Hanahan method (Hanahan, D, J Mol Biol, 166:557-580 (1983)), the electroporation method and the like. In addition, when the host cell is a eukaryotic cell, the vector may be injected into the host cell by microinjection, calcium phosphate precipitation, electroporation, liposome-mediated transfection, DEAE-dextran treatment, gene bombardment and the like.

The method for producing a target protein from the transformed plant may be obtained from the transformed cells, after transforming plant cells with the recombinant vector according to the present invention and culturing the target protein for an appropriate period of time so as to express the target protein. In this case, for the method for expressing the target protein, any method known in the art may be possible.

In addition, as the inventors of the present invention produced a recombinant antigen protein for COVID-19 vaccines from plants, it was possible to obtain glycated spike glycoproteins from the plants. The method for separating and purifying the recombinant antigen protein for the COVID-19 vaccine of the present invention from the transformant or a culture medium thereof may be any method known in the art for separating and purifying a target protein from a plant.

In addition, the present invention provides a method for preparing an antigen protein for preventing or treating COVID-19, including the steps of transforming the recombinant vector according to the present invention into a plant; and separating and purifying a recombinant antigen from the plant.

In addition, the present invention provides a composition for preventing or treating COVID-19, including the antigen produced by the method as an active ingredient.

The pharmaceutical composition for preventing or treating COVID-19 according to the present invention may further include suitable carriers, excipients, and diluents that are commonly used in the preparation of pharmaceutical compositions. The excipient may be, for example, one or more selected from the group consisting of a diluent, a binder, a disintegrant, a lubricant, an adsorbent, a moisturizer, a film-coating material and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates or a preparation for external use, such as plasters, lotions, pastes, sprays, inhalants, patches, sterile injectable solutions or aerosols. The preparation for external use may have a formulation such as creams, gels, patches, sprays, ointments, plasters, lotions, liniments, pastes or cataplasmas.

As the carrier, the excipient and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate and mineral oil may be used..

For formulation, commonly used diluents or excipients such as fillers, thickeners, binders, wetting agents, disintegrants and surfactants are used for preparation.

As the additives of tablets, powders, granules, capsules, pills and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate and Primojel; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine and light anhydrous silicic acid may be used.

As the additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose and sodium carboxymethylcellulose may be used.

In syrups according to the present invention, a white sugar solution, other sugars or sweeteners and the like may be used, and a fragrance, a colorant, a preservative, a stabilizer, a suspending agent, an emulsifier, a viscous agent or the like may be used as necessary.

In emulsions according to the present invention, purified water may be used, and an emulsifier, a preservative, a stabilizer, a fragrance or the like may be used as necessary.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose, HPMC 1828, HPMC 2906, HPMC 2910 and the like may be used, and a surfactant, a preservative, a stabilizer, a colorant, and a fragrance may be used as necessary.

Injections according to the present invention may include solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate and benzene benzoate; co-solvents such as sodium benzoate, sodium salicylate, sodium acetate, urea, urethane, monoethylacetamide, butazolidine, propylene glycol, the Tween series, amide nicotinate, hexamine and dimethylacetamide; buffers such as weak acids and salts thereof (acetic acid and sodium acetate), weak bases and salts thereof (ammonia and ammonium acetate), organic compounds, proteins, albumin, peptone and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂) and ethylenediamine tetraacetic acid; sulfating agents such as 0.1% sodium bisulfide, sodium formaldehyde sulfoxylate, thiourea, disodium ethylenediaminetetraacetate and acetone sodium bisulfite; a pain relief agent such as benzyl alcohol, chlorobutanol, procaine hydrochloride, glucose and calcium gluconate; and suspending agents such as sodium CMC, sodium alginate, Tween 80 and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter+cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75 (S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs and tegester triglyceride matter (TG-95, MA, 57) may be used.

Solid preparations for oral administration include tablets, pills, powders, granules, capsules and the like, and such solid preparations are formulated by mixing the composition with at least one excipient, for example, starch, calcium carbonate, sucrose, lactose, gelatin and the like. In addition to simple excipients, lubricants such as magnesium stearate and talc are also used.

Examples of liquid preparations for oral administration include suspensions, liquids for internal use, emulsions, syrups and the like, and these liquid preparations may include, in addition to simple commonly used diluents, such as water and liquid paraffin, various types of excipients, for example, a wetting agent, a sweetener, a fragrance, a preservative and the like. Preparations for parenteral administration include an aqueous sterile solution, a non-aqueous solvent, a suspension, an emulsion, a freeze-dried preparation and a suppository. Examples of the non-aqueous solvent and the suspension include propylene glycol, polyethylene glycol, a vegetable oil such as olive oil and an injectable ester such as ethyl oleate.

The pharmaceutical composition according to the present invention is administered in a pharmaceutically effective amount. As used herein, the term "the pharmaceutically effective amount" refers to an amount that is sufficient to treat diseases at a reasonable benefit/risk ratio applicable to medical treatment, and an effective dosage level may be determined according to factors including the types of diseases of patients, the severity of disease, the activity of drugs, sensitivity to drugs, administration time, administration route, excretion rate, treatment period, simultaneously used drugs and factors well known in other medical fields.

The pharmaceutical composition according to the present disclosure may be administered as an individual therapeutic agent, may be administered in combination with other therapeutic agents, may be administered sequentially or simultaneously with conventional therapeutic agents, and may be administered once or multiple times. In consideration of all of the above factors, it is important to administer an amount capable of obtaining the maximum effect with a minimum amount without side effects, which can easily be determined by those skilled in the art to which the present invention pertains.

The pharmaceutical composition of the present invention may be administered to a subject by various routes. All modes of administration may be considered, for example, oral administration, subcutaneous injection, intraperitoneal administration, intravenous injection, intramuscular injection, paraspinal space (intrathecal) injection, sublingual administration, buccal administration, rectal insertion, vaginal insertion, ocular administration, otic administration, nasal administration, inhalation, spray through the mouth or nose, dermal administration, transdermal administration and the like.

The pharmaceutical composition of the present invention is determined according to the type of drug as an active ingredient along with several related factors, such as the disease to be treated, route of administration, patient's age, gender, weight and severity of the disease.

As used herein, the term "subject" refers to a subject in need of treatment for a disease, and more specifically, it may refer to a mammal of humans or non-human primates, mice, rats, dogs, cats, horses, cattle and the like, but the present invention is not limited thereto.

As used herein, the term "administration" refers to the provision of a predetermined composition of the present invention to a subject by any suitable method.

As used herein, the term "prevention" refers to all actions that inhibit or delay the onset of a target disease; the term "treatment" refers to all actions that improve or beneficially change a target disease and metabolic abnormalities thereof by the administration of the pharmaceutical composition according to the present invention; and the term "amelioration" refers to all actions that reduce target diseaserelated parameters, for example, the degree of symptoms.

In addition, the present invention provides a kit for preventing or treating COVID-19, including the antigen produced by the method as an active ingredient.

In addition, the present invention provides a method for preventing or treating COVID-19, including the step of administering a composition including the antigen produced by the method as an active ingredient to a non-human animal.

In addition, the present invention provides a vaccine composition for preventing or treating COVID-19, including the vector or the antigen produced by the method as an active ingredient.

As used herein, the term "vaccine" is a biological preparation containing an antigen that causes an immune response in an organism, and refers to an immunogen that induces immunity in an organism by injection or oral administration into a human or animal for the prevention of an infectious disease. The animal is a human or non-human animal, and the non-human animal refers to a pig, a cow, a horse, a dog, a goat, sheep and the like, but the present invention is not limited thereto.

As used herein the term "vaccine composition" may be used by being formulated in the form of an oral formulation such as a powder, granules, a tablet, a capsule, a suspension, an emulsion, a syrup and an aerosol, and a sterile injection solution, according to a typical method. When the composition is prepared, the composition may be prepared using a commonly used diluent or excipient, such as a filler, an extender, a binder, a wetting agent, a disintegrant, and a surfactant. A solid formulation for oral formulation includes a tablet, a pill, a powder, a granule, a capsule and the like, and the solid formulation may be prepared by mixing at least one excipient, for example, starch, calcium carbonate, sucrose or lactose, gelatin and the like with a lecithin-like emulsifier. Further, in addition to simple excipients, lubricants such as magnesium stearate and talc may also be used. As a liquid formulation for oral administration, a suspension, a liquid for internal use, an emulsion, a syrup and the like may be used, and in addition to water and liquid paraffin which are simple commonly used diluents, various excipients, for example, a wetting agent, a sweetener, an aromatic, a preservative and the like, may be included. Examples of a formulation for parenteral administration include an aqueous sterile solution, a non-aqueous solvent, a suspension, an emulsion and a freeze-dried preparation. As the non-aqueous solvent and the suspension, it is possible to use propylene glycol, polyethylene glycol, a vegetable oil such as olive oil, an injectable ester such as ethyl oleate and the like.

The route of administration of the vaccine composition according to the present invention includes, but is not limited to, oral, intravenous, intramuscular, intraarterial, intramedullary, intrathecal, intracardiac, transdermal, subcutaneous, intraperitoneal, intranasal, intestinal, topical, sublingual or rectal routes. Oral or parenteral administration is preferred. As used herein, the term "parenteral" includes subcutaneous, intradermal, intravenous, intramuscular, intraarticular, intrasynovial, intrasternal, intrathecal, intralesional and intracranial injection or infusion techniques. The vaccine composition of the present invention may also be administered in the form of a suppository for rectal administration.

The vaccine composition of the present invention may further include an adjuvant according to usage, and as used herein, the term "adjuvant" refers to a substance or composition that is added to a vaccine or pharmaceutically active ingredients to increase and/or affect an immune response, and it includes a wide range of substances or strategies that can enhance the immunogenicity of an antigen which is incorporated into or co-administered with the adjuvant.

Vaccine adjuvants that are usable with the vaccine adjuvant composition of the present invention may be, for example, aluminum hydroxide, aluminum phosphate, alum (potassium aluminum sulfate), MF59, virosome, AS04 [a mixture of aluminum hydroxide and monophosphoryl lipid A (MPL)], AS03 (a mixture of DL-αtocopherol, squalene and polysorbate 80 as an emulsifier), CpG, flagellin, Poly I:C, AS01, AS02, ISCOMs and ISCOMMATRIX, but the present invention is not limited thereto.

The adjuvant composition according to the present invention may be administered at the same time as the vaccine or at another time, and the frequency of administration of the adjuvant composition may be, for example, daily to every several months, or once or twice before each epidemic season, but the present invention is not limited thereto, and an additional immunization interval may be easily determined by those skilled in the art while examining the course of maintaining immunogenicity.

Hereinafter, preferred examples are presented to aid understanding of the present invention. However, the following examples are provided to more easily understand the present invention, and the content of the present invention is not limited by the following examples.

### [Modes of the Invention]

### [Example]

### Example 1. Preparation of plant-derived recombinant vector expressing COVID-19 variant recombinant spike protein

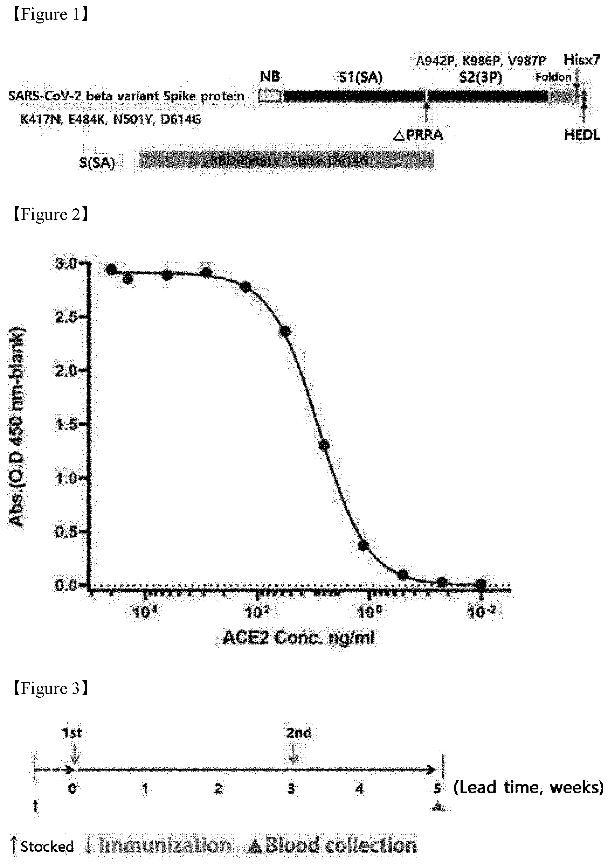

A vector was constructed to express the COVID-19 variant recombinant spike protein in plants. Specifically, K417N, E484K, N501Y D614G mutations to make RBD a beta mutation in the amino acid sequence from glutamine 14 to proline 1162 of the SARS-CoV-2 spike protein (Genbank accession UAQ54630.1) corresponding to the Ancestor were introduced. In order to improve the stability of the spike protein, PRRA, which is the furin cleavage site, was removed, and in order to maintain the spike protein in a prefusion state, three amino acids in the S2 region were substituted with proline (A942P, K986P, V987P). In order to stably form a trimer of the spike protein, the foldon motif of T4 fibritin was fused to the carboxy terminus of proline 1162, and a His-tag consisting of 8 histidines was fused to the carboxy terminus of the foldon motif to separate and purify the spike protein. In order to express the beta mutant spike protein in the plant endoplasmic reticulum, an endoplasmic reticulum signal sequence (NB, Korean Registered Patent No. 10-2138272) was fused at the amino terminus of the spike protein and HDEL, which is an ER retention signal, was fused at the carboxy terminus (the first H is the last histidine of the His-tag, HHHHHHH 'H'DEL), respectively. The gene encoding the SARS-CoV-2 beta mutant spike protein designed in this way was synthesized by codon-optimization to fit *Nicotiana benthamiana,* and the pTEX vector (Korean Patent Application No. 10-2020-0034576) was synthesized by using XbaI and XhoI for cloning. The cleavage map of the recombinant vector prepared by the above method is shown in FIG. 1, and the amino acid sequence and base sequence for each cleavage are as follows.

**[Table 1]**

| **Classification** | **Sequence Number (N→C)** |
|---|---|
| SEQ ID NO: 1 (S1) | Qcvnlttrtqlppaytnsftrgvyypdkvfrssvlhstqdlflpffsnvtwfhaihvsgtngtkrfdnpvlpfndgvyfa steksniirgwifgttldsktqsllivnnatnvvikvcefqfcndpflgvyyhknnkswmesefrvyssannctfeyvs qpflmdlegkqgnfknlrefvfknidgyfkiyskhtpinlvrdlpqgfsaleplvdlpiginitrfqtllalhrsyltpgds ssgwtagaaayyvgylqprtfllkynengtitdavdcaldplsetkctlksftvekgiyqtsnfrvqptesivrfpnitnlc pfgevfnatrfasvyawnrkrisncvadysvlynsasfstfkcygvsptklndlcftnvyadsfvirgdevrqiapgqt gniadynyklpddftgcviawnsnnldskvggnynylyrlfrksnlkpferdisteiyqagstpcngvkgfncyfplq sygfqptygvgyqpyrvvvlsfellhapatvcgpkkstnlvknkcvnfnfngltgtgvltesnkkflpfqqfgrdiadt tdavrdpqtleilditpcsfggvsvitpgtntsnqvavlyqgvnctevpvaihadqltptwrvystgsnvfqtragclig aehvnnsyecdipigagicasyqtqtns |
| SEQ ID NO: 2 (S2) | rsvasqsiiaytmslgaensvaysnnsiaiptnftisvtteilpvsmtktsvdctmyicgdstecsnlllqygsfctqlnra ltgiaveqdkntqevfaqvkqiyktppikdfggfnfsqilpdpskpskrsfiedllfnkvtladagfikqygdclgdiaa rdlicaqkfngltvlppllltdemiaqytsallagtitsgwtfgagaalqipfamqmayrfngigvtqnvlyenqklianq fnsaigkiqdslsstpsalgklqdvvnqnaqalntlvkqlssnfgaissvlndilsrldppeaevqidrlitgrlqslqtyvt qqliraaeirasanlaatkmsecvlgqskrvdfcgkgyhlmsfpqsaphgvvflhvtyvpaqeknfttapaichdgk ahfpregvfvsngthwfvtqrnfyepqiittdntfvsgncdvvigivnntvydplqpeldsfkeeldkyfknhtsp |
| SEQ ID NO: 3 (Foldon) | gyipeaprdgqayvrkdgewvllstfl |
| SEQ ID NO: 4 (GS-linker) | gsgs |
| SEQ ID NO: 5 (x7 his-tag) | hhhhhhh |
| SEQ ID NO: 6 (HDEL) | hdel |
| SEQ ID NO: 7 (Full amino acid sequence (excluding NB)) | gsqcvnlttrtqlppaytnsftrgvyypdkvfrssvlhstqdlflpffsnvtwfhaihvsgtngtkrfdnpvlpfndgvy fasteksniirgwifgttldsktqsllivnnatnvvikvcefqfcndpflgvyyhknnkswmesefrvyssannctfey vsqpflmdlegkqgnfknlrefvfknidgyfkiyskhtpinlvrdlpqgfsaleplvdlpiginitrfqtllalhrsyltpg dsssgwtagaaayyvgylqprtfllkynengtitdavdcaldplsetkctlksftvekgiyqtsnfrvqptesivrfpnit nlcpfgevfnatrfasvyawnrkrisncvadysvlynsasfstfkcygvsptklndlcftnvyadsfvirgdevrqiap gqtgniadynyklpddftgcviawnsnnldskvggnynylyrlfrksnlkpferdisteiyqagstpcngvkgfncyf plqsygfqptygvgyqpyrvvvlsfellhapatvcgpkkstnlvknkcvnfnfngltgtgvltesnkkflpfqqfgrdi adttdavrdpqtleilditpcsfggvsvitpgtntsnqvavlyqgvnctevpvaihadqltptwrvystgsnvfqtragc ligaehvnnsyecdipigagicasyqtqtnsrsvasqsiiaytmslgaensvaysnnsiaiptnftisvtteilpvsmtkt svdctmyicgdstecsnlllqygsfctqlnraltgiaveqdkntqevfaqvkqiyktppikdfggfnfsqilpdpskps krsfiedllfnkvtladagfikqygdclgdiaardlicaqkfngltvlppllltdemiaqytsallagtitsgwtfgagaalqi pfamqmayrfngigvtqnvlyenqklianqfnsaigkiqdslsstpsalgklqdvvnqnaqalntlvkqlssnfgais svlndilsrldppeaevqidrlitgrlqslqtyvtqqliraaeirasanlaatkmsecvlgqskrvdfcgkgyhlmsfpqs aphgvvflhvtyvpaqeknfttapaichdgkahfpregvfvsngthwfvtqrnfyepqiittdntfvsgncdvvigiv nntvydplqpeldsfkeeldkyfknhtspgyipeaprdgqayvrkdgewvllstflgsgshhhhhhhhdel |

**[Table 2]**

| **Classification** | **Sequence Number (5'→3')** |
|---|---|
| SEQ ID NO: 8 (S1) | cagtgtgttaaccttaccaccagaactcagttacccccagcatacactaattctttcacacgtggtgtttactaccctgacaa agttttcagaagcagcgttttacacagcactcaggatttattcctacctttcttttccaacgtgacctggttccatgctatacatg tatctgggaccaatggtaccaagaggtttgataacccggtcctaccatttaatgatggagtctattttgcctccactgagaag tctaatataataagaggctggatttttggaactactcttgattcgaagacccagagtctacttattgttaataacgctacaaatg ttgttatcaaagtatgtgaatttcaattctgtaatgatccattcttgggtgtttactaccacaaaaacaacaaaagttggatgga aagtgagtttcgggtttatagcagtgcgaataattgcacttttgagtacgtctcccaaccttttcttatggaccttgaaggaaa gcagggaaatttcaagaatcttcgcgaatttgtgtttaagaatatcgatggttatttcaagatatattctaagcacacgcctatt aatttagtgcgagatctccctcagggtttttcggcgctggaaccattggtagatttgccgataggaatcaatatcactaggtt ccagactttacttgctctgcatagaagttacttgacccctggagatagctcatcaggttggacagctggtgcggcagcttat tacgtggggtatcttcagcctaggacgttcctattaaaatataatgaaaatggaaccattacagatgctgtagactgtgcact tgaccctctctcagaaacaaagtgtacgttgaaatccttcacggtagaaaaagggatctaccaaacgtctaacttcagagt ccagccaacagaatctattgtgagatttcccaatattacaaacttgtgccctttcggagaagtttttaacgccaccaggtttgc atcggtttatgcttggaacaggaaaagaatcagcaactgtgttgctgattatagtgtcctatataactccgcatccttttccact ttcaagtgttacggagtttctcctactaaattaaatgatctctgctttactaatgtctatgcagattcatttgtaatcagaggtgat gaggtcagacaaatcgctccagggcagactggaaacattgctgattataattataagcttcctgatgattttacaggctgcg ttatagcatggaattctaataatcttgactctaaggtggggggaaattataattacctgtatagactgtttaggaagagcaatc tcaagcctttcgagagagacatttcaactgagatctaccaggcgggaagcactccgtgtaatggtgttaagggttttaattg ttactttcctttacagtcatacggtttccaacccacgtatggggttggttaccaaccgtaccgagtagtagtactttctttcgag cttctacatgccccagcaactgtttgtggacctaagaagtctactaatttggttaaaaataagtgtgtcaattttaatttcaatg gacttacgggcacaggagttcttactgagtctaacaagaagtttctgcctttccagcagttcggcagagatattgctgacac tactgatgctgtgcgtgatccacagacacttgaaattcttgacattacaccatgttcttttggtggcgtgagtgttataactccc ggaacaaatacctccaaccaggtggctgttctgtatcagggtgtgaactgtacagaagtccctgttgcaattcatgcagat cagcttactcctacctggcgtgtttattctacgggttccaatgtttttcaaacacgtgcaggctgcttgataggggctgaacat gtcaacaactcatatgaatgcgacatacccataggtgcaggtatatgcgctagttatcagactcagaccaattct |
| SEQ ID NO: 9 (S2) | cgaagtgtagctagtcaatccatcattgcctacactatgtcacttggtgcagaaaattcagttgcttactctaataactctattg ccatacccacaaattttactattagtgttaccacagaaattctaccagtgtctatgaccaagacatcagttgactgtacaatgt atatttgcggggattcaactgagtgctcgaatctgttgttgcaatacggcagtttttgtacccaattgaaccgggctctgact ggaatagctgtggaacaagataaaaacacccaagaagtttttgcacaagtcaaacaaatttataaaacaccaccaattaaa gatttcggtggtttcaacttctcacaaatactgccagatccgagcaaaccaagcaagaggtcattcattgaagacctactttt caacaaagtgacacttgcagatgctggcttcattaaacagtatggtgattgcttgggggatattgctgctagagacctcattt gtgcacaaaagtttaacgggctgacagtgttgccacctttgttgacagatgagatgattgctcagtacacttctgcactgctc gctggtacaatcacatctgggtggacctttggtgcaggtgctgccttacaaataccatttgctatgcagatggcttataggtt |
| | caatggtatcggagttacacagaacgttctctatgagaaccaaaaattgattgccaaccaattcaatagtgccattggcaag attcaggactcactttcaagcacacctagtgcacttggaaagttgcaagatgtggtcaaccagaatgcacaagctttaaac acgcttgtgaaacaactcagctccaactttggggcaatttcaagtgttttgaatgatatcctttcacgtcttgatccaccagaa gccgaggtgcaaattgacaggttgatcacaggccgacttcaaagtttgcagacttatgtgactcaacaattaattagggca gcagaaatccgcgcttcggctaatctggcggctactaaaatgtcagagtgtgtacttggacaatctaaacgagttgattttt gcggaaagggctatcatctcatgtccttccctcagtcagcgcctcacggtgtagtgttcttgcacgtgacttacgttcctgca caagaaaagaatttcacaactgctccggccatttgtcatgatggaaaagcccactttccgcgtgaaggtgtctttgtttcga atggcacacactggtttgtaacccaaaggaatttttatgagccacaaatcattacgacggacaacacttttgtgtctggtaat tgtgatgttgtaatcggaatcgtcaacaacaccgtttacgatcctttgcagcctgagttagattctttcaaagaggagctgga taagtatttcaagaatcatacatcaccc |
| SEQ ID NO: 10 (NB(New BiP) | atggctcgctcgtttggagctaacagtaccgttgtgttggcgatcatcttcttcggtgagtgattttccgatcttcttctccgatt tagatctcctctacattgttgcttaatctcagaaccttttttcgttgttcctggatctgaatgtgtttgtttgcaatttcacgatctta aaaggttagatctcgattggtattgacgattggaatctttacgatttcaggatgtttatttgcgttgtcctctgca ggatcc |
| SEQ ID NO: 11 (Foldon) | ggttatattcctgaggctcctagagatgggcaggcttacgttcgtaaagatggcgaatgggttttgctttctacttttttg |
| SEQ ID NO: 12 (GS-linker) | ggatctggttca |
| SEQ ID NO: 13 (×7 his-tag) | caccaccatcaccaccatcac |
| SEQ ID NO: 14 (HDEL) | catgatgagctc |
| SEQ ID NO: 15 (total nucleotide sequence) | tctagaattattacatcaaaacaaaaaatggctcgctcgtttggagctaacagtaccgttgtgttggcgatcatcttcttcggt gagtgattttccgatcttcttctccgatttagatctcctctacattgttgcttaatctcagaaccttttttcgttgttcctggatctga atgtgtttgtttgcaatttcacgatcttaaaaggttagatctcgattggtattgacgattggaatctttacgatttcaggatgtttat ttgcgttgtcctctgcaggatcccagtgtgttaaccttaccaccagaactcagttacccccagcatacactaattctttcacac gtggtgtttactaccctgacaaagttttcagaagcagcgttttacacagcactcaggatttattcctacctttcttttccaacgt gacctggttccatgctatacatgtatctgggaccaatggtaccaagaggtttgataacccggtcctaccatttaatgatgga gtctattttgcctccactgagaagtctaatataataagaggctggatttttggaactactcttgattcgaagacccagagtcta cttattgttaataacgctacaaatgttgttatcaaagtatgtgaatttcaattctgtaatgatccattcttgggtgtttactaccaca aaaacaacaaaagttggatggaaagtgagtttcgggtttatagcagtgcgaataattgcacttttgagtacgtctcccaacc ttttcttatggaccttgaaggaaagcagggaaatttcaagaatcttcgcgaatttgtgtttaagaatatcgatggttatttcaag atatattctaagcacacgcctattaatttagtgcgagatctccctcagggtttttcggcgctggaaccattggtagatttgccg ataggaatcaatatcactaggttccagactttacttgctctgcatagaagttacttgacccctggagatagctcatcaggttg gacagctggtgcggcagcttattacgtggggtatcttcagcctaggacgttcctattaaaatataatgaaaatggaaccatt acagatgctgtagactgtgcacttgaccctctctcagaaacaaagtgtacgttgaaatccttcacggtagaaaaagggatc taccaaacgtctaacttcagagtccagccaacagaatctattgtgagatttcccaatattacaaacttgtgccctttcggaga agtttttaacgccaccaggtttgcatcggtttatgcttggaacaggaaaagaatcagcaactgtgttgctgattatagtgtcct atataactccgcatccttttccactttcaagtgttacggagtttctcctactaaattaaatgatctctgctttactaatgtctatgca gattcatttgtaatcagaggtgatgaggtcagacaaatcgctccagggcagactggaaacattgctgattataattataagc ttcctgatgattttacaggctgcgttatagcatggaattctaataatcttgactctaaggtggggggaaattataattacctgta tagactgtttaggaagagcaatctcaagcctttcgagagagacatttcaactgagatctaccaggcgggaagcactccgt gtaatggtgttaagggttttaattgttactttcctttacagtcatacggtttccaacccacgtatggggttggttaccaaccgta ccgagtagtagtactttctttcgagcttctacatgccccagcaactgtttgtggacctaagaagtctactaatttggttaaaaat aagtgtgtcaattttaatttcaatggacttacgggcacaggagttcttactgagtctaacaagaagtttctgcctttccagcag ttcggcagagatattgctgacactactgatgctgtgcgtgatccacagacacttgaaattcttgacattacaccatgttctttt ggtggcgtgagtgttataactcccggaacaaatacctccaaccaggtggctgttctgtatcagggtgtgaactgtacagaa gtccctgttgcaattcatgcagatcagcttactcctacctggcgtgtttattctacgggttccaatgtttttcaaacacgtgcag gctgcttgataggggctgaacatgtcaacaactcatatgaatgcgacatacccataggtgcaggtatatgcgctagttatc agactcagaccaattctcgaagtgtagctagtcaatccatcattgcctacactatgtcacttggtgcagaaaattcagttgctt actctaataactctattgccatacccacaaattttactattagtgttaccacagaaattctaccagtgtctatgaccaagacatc agttgactgtacaatgtatatttgcggggattcaactgagtgctcgaatctgttgttgcaatacggcagtttttgtacccaattg aaccgggctctgactggaatagctgtggaacaagataaaaacacccaagaagtttttgcacaagtcaaacaaatttataaa acaccaccaattaaagatttcggtggtttcaacttctcacaaatactgccagatccgagcaaaccaagcaagaggtcattc attgaagacctacttttcaacaaagtgacacttgcagatgctggcttcattaaacagtatggtgattgcttgggggatattgct gctagagacctcatttgtgcacaaaagtttaacgggctgacagtgttgccacctttgttgacagatgagatgattgctcagt acacttctgcactgctcgctggtacaatcacatctgggtggacctttggtgcaggtgctgccttacaaataccatttgctatg cagatggcttataggttcaatggtatcggagttacacagaacgttctctatgagaaccaaaaattgattgccaaccaattca atagtgccattggcaagattcaggactcactttcaagcacacctagtgcacttggaaagttgcaagatgtggtcaaccaga atgcacaagctttaaacacgcttgtgaaacaactcagctccaactttggggcaatttcaagtgttttgaatgatatcctttcac |
| | gtcttgatccaccagaagccgaggtgcaaattgacaggttgatcacaggccgacttcaaagtttgcagacttatgtgactc aacaattaattagggcagcagaaatccgcgcttcggctaatctggcggctactaaaatgtcagagtgtgtacttggacaat ctaaacgagttgatttttgcggaaagggctatcatctcatgtccttccctcagtcagcgcctcacggtgtagtgttcttgcac gtgacttacgttcctgcacaagaaaagaatttcacaactgctccggccatttgtcatgatggaaaagcccactttccgcgtg aaggtgtctttgtttcgaatggcacacactggtttgtaacccaaaggaatttttatgagccacaaatcattacgacggacaac acttttgtgtctggtaattgtgatgttgtaatcggaatcgtcaacaacaccgtttacgatcctttgcagcctgagttagattcttt caaagaggagctggataagtatttcaagaatcatacatcacccggttatattcctgaggctcctagagatgggcaggctta cgttcgtaaagatggcgaatgggttttgctttctacttttttgggatctggttcacaccaccatcaccaccatcaccatgatga gctctagctcgag |

### Example 2. Confirmation of biological activity of recombinant antigen (S(rBeta3P)) according to COVID-19 variant recombinant spike protein expression vector

In order to confirm the biological activity of the antigen prepared by the COVID-19 variant recombinant spike protein expression vector of Example 1, the binding characteristics of ACE2 were analyzed. Specifically, after preparing a recombinant antigen (S(rBeta3P)) from the recombinant vector of Example 1, it was diluted in PBS, fixed at 100 ng per well and treated at 4°C for o/n (16 hours). Afterwards, the antigen-immobilized wells were blocked in 1x ELISA assay buffer (Invitrogen, DS98200) at room temperature for 1 hour, and hACE2 (SinoBiological, 10108-H05H) was respectively added at 0.01024, 0.0512, 0.256, 1.28, 6.4, 32, 160, 800, 4000, 2000, and 40000 ng/Ml to react at room temperature for one hour. In addition, for the second antibody treatment, a-mouse lgG (Bethyl, A90-146P) was added at a ratio of 1: 5,000 at room temperature for 1 hour to react. Finally, the color reaction was induced for 10 minutes by adding 50% TMB substrate, and after the reaction was terminated by treatment with 4N H₂SO₄ stop solution, the absorbance (OD) of each well at a wavelength of 450 nm was measured by using an ELISA reader.

As a result, it was confirmed that the hACE2 protein formed a concentration-dependent binding to the recombinant antigen (S(rBeta3P)) (FIG. 2), and after applying a non-linear regression fit by using the GraphPad Prism 9 statistical program, 50% binding concentration was calculated, and as a result, it was measured that EC₅₀ = 7.843 ng/mL

### Example 3. Confirmation of immunogenicity against Wuhan virus

In order to confirm the immunogenicity of the antigen prepared by the COVID-19 variant recombinant spike protein expression vector of Example 1, the protein was injected into mice as shown in FIG. 3 to obtain serum, and with this serum, the Plaque Reduction Neutralization Test (PRNT) was performed for Wuhan and beta-mutant strains, respectively.

First of all, in order to obtain serum from mice, the recombinant antigen (S(rBeta3P)) prepared according to Example 1 was quantified by measuring UV₂₈₀ absorbance. Thereafter, test vaccines G1 to G5 were prepared according to Table 3 below. G1 to G3 were prepared by diluting the quantified proteins in purified water and then mixing the same with an immune enhancer (Eyegene Co., Ltd.) at a ratio of 1: 1, and a PBS solution was used as a control group (G5). A total of 100 µL of 50 µL each of G1 to G5 was primarily injected into the left and right femoral muscles of 6-week-old female Balb/c mice (Groups 1 to 5), respectively, and on the third week, the same amount of protein was secondarily injected, and after the secondary injection, serum was obtained on the fifth week after 2 weeks (FIG. 3).

**[Table 3]**

| **Group** | **Test vaccine group** | | | **Antigen-only administration group** | **Control group** |
|---|---|---|---|---|---|
| | **G1** | **G2** | **G3** | **G4** | **G5** |
| **Ag(µg)/dose** | 5 | 10 | 25 | 25 | -(PBS) |
| **Adjuvant** | + | + | + | - | - |

Afterwards, in order to perform PRNT, Vero E6 cells (ATCC) were suspended at 2.5 × 10⁵/mL, and then dispensed by 1 mL into a 12-well plate (2.5 × 10⁵ cells/1 mL/well/12-well plate), and the 12-well plate in which the cells were dispensed was cultured for 24 hours in a CO₂ incubator at 37°C. The serum sample to be used in the experiment was non-assimilated at 56°C for 30 minutes, and the non-assimilated sample serum was diluted in a stepwise manner from 1/10 to 2-fold nu using 2% FBS + 1% Pen/Strep + DMEM. Thereafter, the COVID-19 virus was diluted from the stock solution to 6 × 10³ PFU/mL, and the diluted serum was dispensed at a concentration of about 50 PFU (so that 30 to 60 plaques were observed). The diluted serum was incubated for 1 hour in a CO₂ incubator, and after removing the supernatant of vero cells that were prepared in advance, 200 µL of the serum and virus mixture was dispensed. The plate to which the serum and virus mixture was dispensed was incubated for 1 hour in a CO₂ incubator, and the plate was shaken well every 10 minutes during incubation to mix, and then, the serum and virus mixture was removed. Meanwhile, overlay media was prepared with 4% FBS MEM (2X) and 1.5% agar at a ratio of 1:1. After dispensing 1 mL of the prepared overlay media, it was incubated for 3 days in a CO₂ incubator. In addition, the Crystal Violet Mixture (based on 500 mL) which was prepared from 33 mL of the crystal violet solution, 108 m: of 37% formaldehyde solution and 25 mL of EtOH + 334 mL of DW was prepared, and after dispensing the same and staining, the staining reagent and agar were removed, and then, it was sufficiently dried at room temperature, and the number of plaques was counted. Experiments were performed for all samples by repeating two times, and a negative test group (NC, virus and serum samples not included) and a positive control group (virus only included) were included.

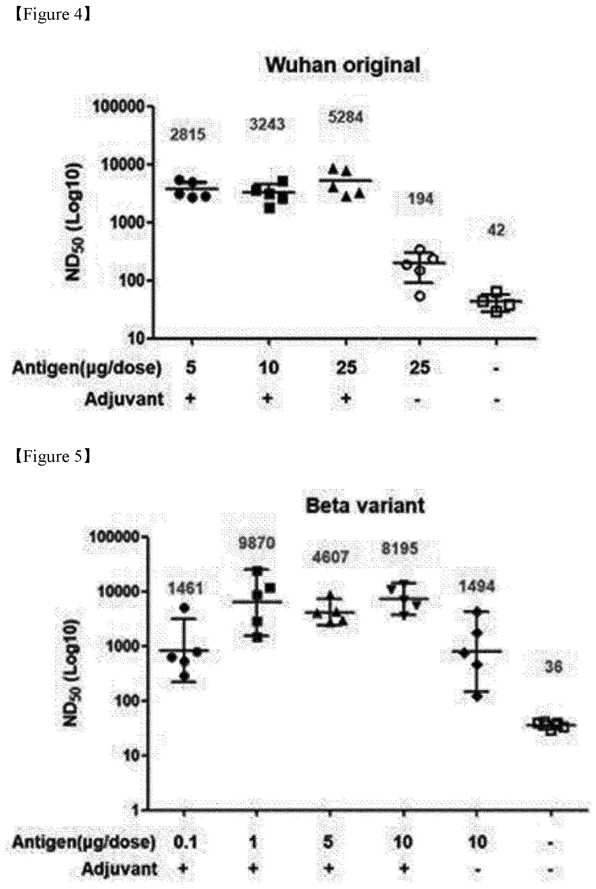

As a result, the neutralizing antibodies (ND₅₀) against the Wuhan strain in the test vaccine G1 (5 µg/dose), G2 (10 µg/dose) and G3 (25 µg/dose) administration groups including the adjuvant were respectively measured at 2,815, 3,243 and 5,284 (FIG. 4), and the neutralizing antibody titer of the antigen-only group administration group not including the adjuvant was measured at 194 (G4) against the Wuhan virus. It was confirmed that the immunogenicity was remarkably excellent, because the neutralizing antibody titers for each of the Wuhan virus strains of the test vaccines (G1 to G3) including the adjuvant corresponded to about 15 to 27 times that of the test vaccine (G4) without the adjuvant.

### Example 4. Confirmation of immunogenicity of recombinant antigen (S(rBeta3P)) against beta/delta/omicron virus

In order to confirm the immunogenicity of the antigen prepared by the COVID-19 variant recombinant spike protein expression vector of Example 1 against the beta/delta/omicron virus, the Plaque Reduction Neutralization Test (PRNT) was performed, respectively. The experiment was performed in the same manner as in Example 3 except that the concentration of the recombinant antigen (S(rBeta3P)) of the present invention was prepared and administered according to Table 4.

**[Table 4]**

| **Group** | **Test vaccine group** | | | | **Antigen-only administration group** | | **Control group** |
|---|---|---|---|---|---|---|---|
| | **G1** | **G2** | **G3** | **G4** | **G5** | **G6** | **G7** |
| **Ag(µg)/dose** | 0.1 | 1 | 5 | 10 | 25 | 10 | - (PBS) |
| **Adjuvant** | + | + | + | + | - | - | - |

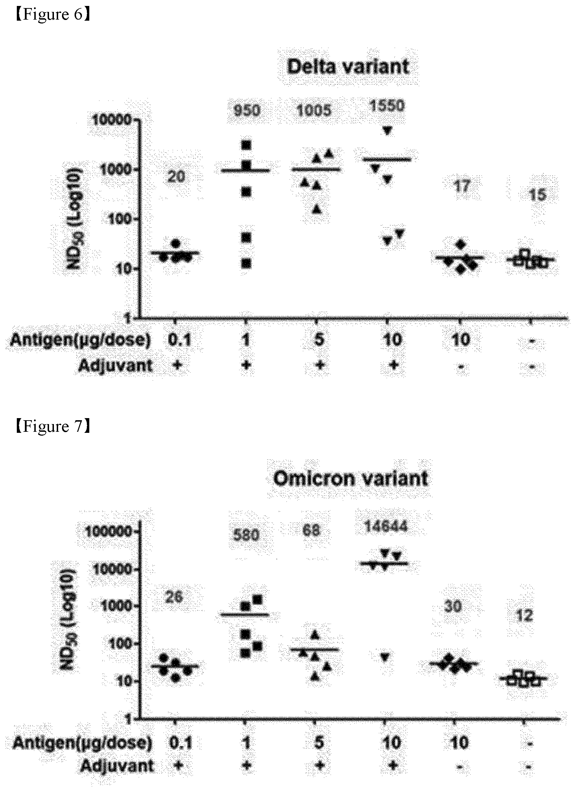

As a result, the result values for the beta-mutated virus strains in the test vaccine G1 (0.1 µg/dose), G2 (1 µg/dose), G3 (5 µg/dose) and G4 (10 µg/dose) administration groups including the adjuvant were measured as 1,461, 9,870, 4,607, 8,195 and 1,494, respectively (FIG. 5). The neutralizing antibodies (ND₅₀) against the delta-mutated strain were measured as 20, 950, 1,005, 1,550 and 17, respectively (FIG. 6), and the result values for the omicron-mutated strain were measured as 26, 580, 68, 14,644 and 30, respectively (FIG. 7). When the serum of the present invention is administered together with an immune enhancer, it was confirmed that only a small amount of the reagent produces an immune effect, because the neutralizing antibody titers against the delta and omicron virus strains were all measured as low as ND₅₀ = 32 or less in the antigen-only groups (G5 and G6).

### Example 5. Confirmation of immunogenicity of recombinant antigen (S(rBeta3P)) according to anti-Spike IgG ELISA test

Anti-Spike IgG ELISA was performed on the immunogenicity of the antigen prepared by the COVID-19 variant recombinant spike protein expression vector of Example 1, respectively.

Specifically, after preparing serum in the same manner as in Example 3 according to Table 5, 100 ng of the recombinant antigen (S(rBeta3P)) was immobilized per well and treated at 4°C for o/n (about 16 hours), and afterwards, the antigen-immobilized wells were blocked with 3% skim milk in PBS-T at 37°C for 1 hour. For the primary antibody treatment, the serum obtained 2 weeks after the secondary administration of the recombinant antigen (S(rBeta3P)) to the mice was diluted to 1/150 to 1/11,718,750 and reacted at 37°C for 2 hours. For secondary antibody treatment, anti-Mouse IgG was diluted by 1/5,000 and reacted at 37°C for 1 hour. Finally, TMB substrate was added to induce a color reaction for 10 minutes, and after treating 4N H₂SO₄ stop solution to terminate the reaction, the absorbance (OD) of each well was measured at a wavelength of 450 nm by using an ELISA reader. In this case, the cut off was set to 0.2 (4 times the PBS endpoint).

**[Table 5]**

| **Group** | **Test vaccine group** | | | | **Antigen-only administration group** |
|---|---|---|---|---|---|
| | **G1** | **G2** | **G3** | **G4** | **G5** |
| **Ag(µg)/dose** | 0.1 | 1 | 5 | 10 | 10 |
| **Adjuvant** | + | + | + | + | - |

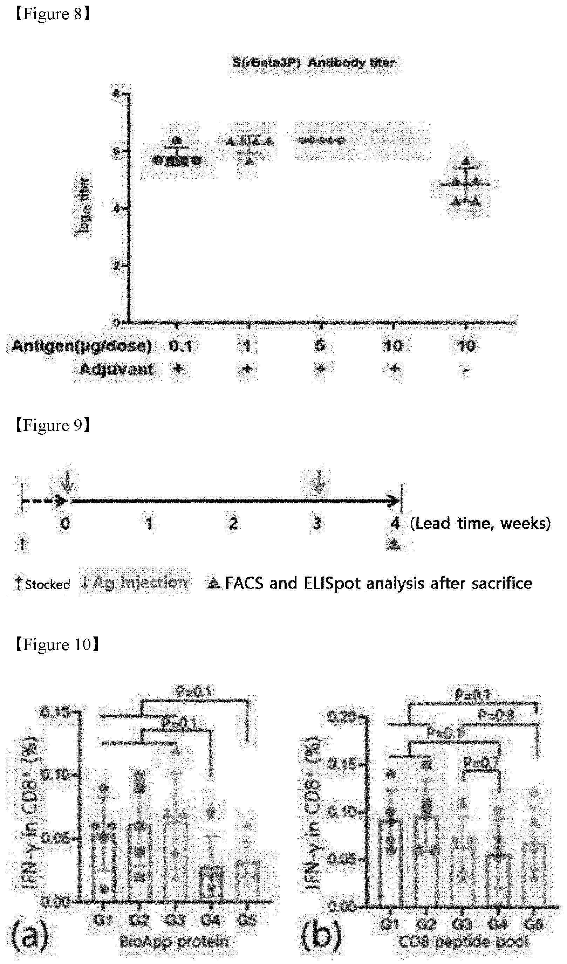

As a result, as shown in FIG. 10, in the test vaccine G1 (0.1 µg/dose), G2 (1 µg/dose), G3 (5 µg/dose) and G4 (10 µg/dose) administration group including the adjuvant, the concentration-dependent antigen-specific antibody titers of 5.81, 6.23, 6.37 and 6.37 logic titers were confirmed. The antigen-specific antibody titer of the serum of the G5 (10 µg/dose) administration group without the adjuvant was rather low at 4.83 log₁₀ titer, but it was confirmed that it showed an excellent antibody titer level.

### Example 6. Confirmation of immunogenicity of recombinant antigen (S(rBeta3P)) according to intracellular cytokine staining test

In order to confirm the immunogenicity of the antigen prepared by the COVID-19 variant recombinant spike protein expression vector of Example 1, the cytokine analysis (cellular staining-cytokines) of mouse spleen CD4⁺ and CD8⁺ T cells was performed.

First of all, serum was prepared in the same manner as in Example 3 according to Table 6, and then, the spleen was removed by inoculating the mouse according to FIG. 9. In addition, the analysis of T cells induced by the cytokine (INF-γ, TNF-α) was performed, and for stimulation, inoculation antigen protein for confirming humoral immunity or CD8-specific peptide pool for confirming cellular immunity was used. Stimulation was performed at 500 ng/well for the protein and 4 µg/well for the peptide, and in this case, stimulation was performed for 16 hours, and T cells induced by the cytokine were treated with Golgi treatment for 12 hours after stimulation time of 16 hours and stimulation time of 4 hours.

**[Table 6]**

| **Group** | **Test vaccine group** | | | **Antigen-only administration group** | **Control group** |
|---|---|---|---|---|---|
| | **G1** | **G2** | **G3** | **G4** | **G5** |
| **Ag(µg)/dose** | 1 | 5 | 10 | 10 | -(PBS) |
| **Adjuvant** | + | + | + | - | - |

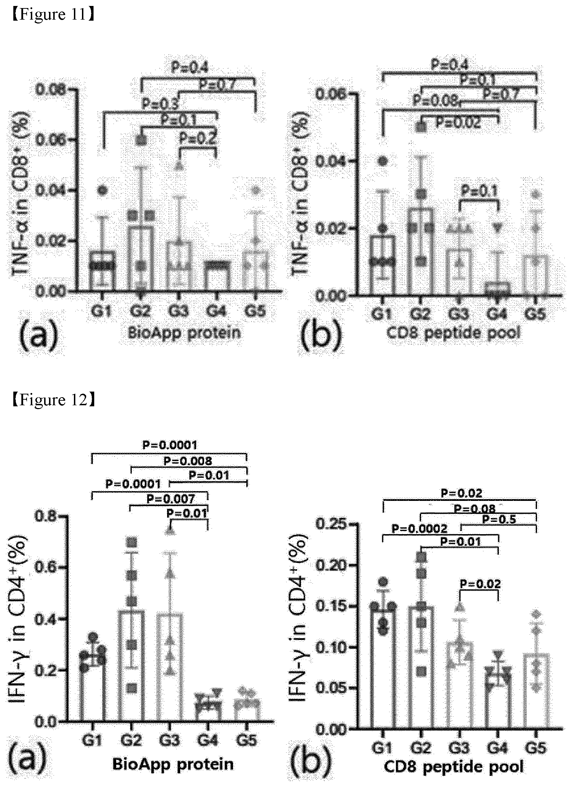

As a result, an increasing tendency in CD8⁺ T cells expressing INF-γ, which is the core of cellular immunity, was confirmed in the splenocytes of the vaccine administration group (FIG. 10(a)), but the result was not statistically significant (p = 0.1). Even when stimulated with the CD8 peptide pool, no significant increase in INF-γ CD8⁺ T cells was confirmed (FIG. 10(b)), and CD8⁺ T cells expressing TNF-α were also not significantly different (FIG. 11(a) and FIG. 11(b)) (in the present invention, (a) in the drawing means the graph on the left, and (b) means the graph on the right in the drawing.).

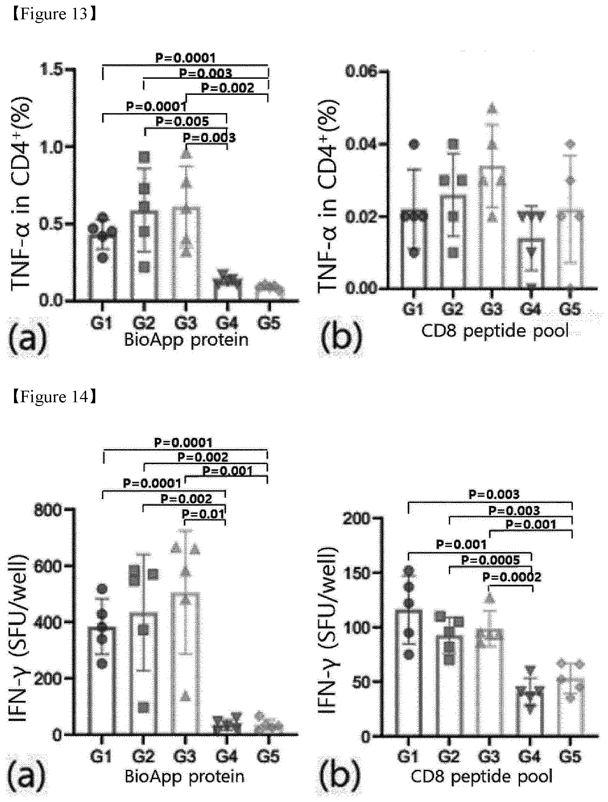

On the other hand, as a result of CD4⁺ T cell cytokine staining involved in humoral immunity, a significant increase was confirmed when it was stimulated with the inoculated antigen (FIGS. 12 (a) and 13 (a)). Specifically, INF-γ CD4⁺ T cells in the splenocytes of the test vaccine administration groups (G1 to G3) compared to the control group (G5) increased in a concentration-dependent manner (p value = 0.01 or less) (FIG. 12 (a)), and TNF-α CD4⁺ T cells also showed the same pattern (p value = 0.003 or less) (FIG. 13(a)). However, when it was stimulated with a CD8 peptide pool that specifically stimulates CD8⁺ T cells, an increase in CD4⁺ T cells expressing INF-γ or TNF-α was not confirmed compared to the control group in G1 to 3 (FIG. 12(b) and FIG. 13(b)).

### Example 7. Confirmation of immunogenicity of recombinant antigen (S (rBeta3P)) according to ELISpot-cytokines test

In order to confirm the immunogenicity of the antigen prepared by the COVID-19 variant recombinant spike protein expression vector of Example 1, ELISpot (Enzyme-Linked ImmunoSpot) analysis was performed on splenocytes. Specifically, splenocytes were prepared in the same manner as in Example 6, and ELISpot analysis for INF-γ and IL-4 was performed, and splenocytes were stimulated by using an inoculation antigen protein (Bioapp protein) to confirm humoral immunity or a CD8-specific peptide pool to confirm cellular immunity. The antibodies against INF-γ and IL-4 were coated on PVDF (polyvinylidene fluoride)-backed microplates and ELISpot plates. Thereafter, cells were added, and as an antigen to stimulate the cells, the antigen protein (BioApp protein) or SARS-2 CD8 epitope prediction peptide pool was added at 500 ng/well and 4 µg/well, respectively, for 48 hours. After culturing, cells were removed, and detection Ab which was directly coupled to the enzyme or coupled to biotin was added, and Streptavidin-enzyme which was directly coupled with enzyme was added. As a result, the number of color spots formed in cells secreting cytokines was counted to confirm the number of cells responding to the stimulating antigen.

As a result, it was confirmed that the INF-γ of the splenocytes of the test vaccine administration groups G1 to G3 that were stimulated with the inoculation antigen increased significantly compared to the control group (G5) (p value = 0.002 or less), and the control group (G4) which was inoculated with only the antigen showed an INF-γ expression level similar to that of the control group (FIG. 14(a)). When it was stimulated by using the CD8 peptide pool, the INF-γ expression level of the test vaccine administration group G1 to G3 splenocytes increased significantly (p value = 0.003 or less) (FIG. 14(b)).

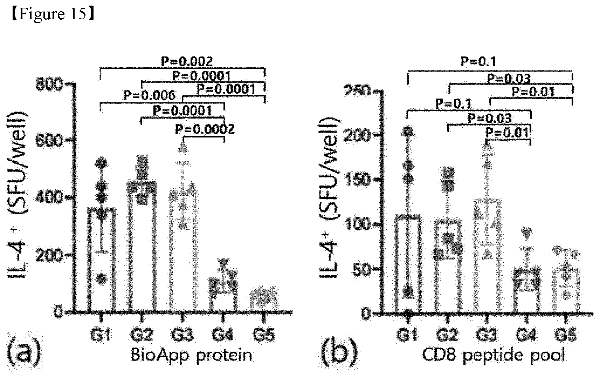

In addition, the IL-4⁺ expression of the test vaccine administration group G1 to G3 splenocytes that were stimulated with the inoculation antigen significantly increased (p value = 0.002 or less) compared to the control group (G5) (FIG. 15 (a)), but in the test vaccine administration group G1 which was stimulated by using the CD8 peptide pool, there was no statistically significant difference compared to the control group (G5) (p value = 0.1) (FIG. 15 (b)). However, in the case of G2 and G3, as the p values were analyzed to be 0.03 and 0.01, respectively (FIG. 15(b)), it was confirmed that IL-4 expression by CD8 stimulation significantly increased compared to the PBS administration group (G5) as the concentration of the administered antigen was increased.

The above description of the present invention is for illustrative purposes, and those skilled in the art will understand that it can be easily modified into other specific forms without changing the technical spirit or essential features of the present invention. Therefore, it should be understood that the exemplary embodiments described above are illustrative in all respects and not restrictive.

### [Industrial Applicability]

In the plant-based COVID-19 variant recombinant spike protein expression vector and the recombinant protein using the expression vector according to the present invention, a plant expression system is used such that the recombinant protein does not have the disadvantages of animal-derived recombinant proteins and exhibits an excellent SARS coronavirus 2 prevention and treatment effect, and thus can be effectively utilized as a safe composition for preventing and treating COVID-19.

## Claims

1. A recombinant vector for an antigen protein for preventing or treating COVID-19, wherein in a polynucleotide encoding an S1 subunit of the spike glycoprotein represented by the amino acid sequence of SEQ ID NO: 1, the S1 subunit has a mutation at one or more positions selected from the group consisting of positions 417, 484, 501 and 614 from the N-terminus of the amino acid sequence.

2. The recombinant vector of claim 1, wherein the recombinant vector further comprises a polynucleotide encoding an S2 subunit of the spike glycoprotein represented by the amino acid sequence of SEQ ID NO: 2, and
wherein the S2 subunit has a mutation at one or more positions selected from the group consisting of positions 942, 986 and 987 from the N-terminus of the amino acid sequence.

3. The recombinant vector of claim 1, wherein the mutation is any one or more mutations selected from the group consisting of K417N, E484K, N501Y and D614G.

4. The recombinant vector of claim 2, wherein the mutation is any one or more mutations selected from the group consisting of A942P, K986P and V987P.

5. The recombinant vector of claim 1, wherein the recombinant vector further comprises a polynucleotide encoding the new chaperone binding protein (New BiP; NB) protein.

6. The recombinant vector of claim 1, wherein the recombinant vector further comprises a polynucleotide encoding the foldon (Fd) protein.

7. The recombinant vector of claim 1, wherein the recombinant vector further comprises a polynucleotide encoding the HDEL protein.

8. The recombinant vector of claim 1, wherein the recombinant vector further comprises a polynucleotide encoding an antibody in which the furin cleavage site (PRRA) between the S1 subunit and the S2 subunit is deleted.

9. The recombinant vector of claim 1, wherein the recombinant vector is expressed in a plant.

10. A transformant, which is transformed with the recombinant vector according to any one of claims 1 to 9.

11. The transformant of claim 10, wherein the transformant is a plant.

12. A method for preparing an antigen protein for preventing or treating COVID-19, comprising the steps of:
transforming the recombinant vector of claim 1 into a plant; and
separating and purifying a recombinant antigen from the plant.

13. A composition for preventing or treating COVID-19, comprising the antigen produced by the method of claim 12 as an active ingredient.

14. A kit for preventing or treating COVID-19, comprising a composition comprising the antigen produced by the method of claim 12 as an active ingredient and instructions.

15. A method for preventing or treating COVID-19, comprising the step of:
administering a composition comprising the antigen produced by the method of claim 12 as an active ingredient to a non-human animal.

16. A vaccine composition for preventing or treating COVID-19, comprising the vector of claim 1 or the antigen produced by the method of claim 12 as an active ingredient.

17. Use of a composition comprising the vector of claim 1 or the antigen produced by the method of claim 12 as an active ingredient in the prevention or treatment of COVID-19.

18. Use of a composition comprising the vector of claim 1 or the antigen produced by the method of claim 12 as an active ingredient in the preparation of a medicament for treating COVID-19.
